# EUROPEAN PATENT APPLICATION

(11) **EP 4 749 627 A1**
(43) Date of publication of application: **27.05.2026**
(21) Application number: 24844715.3
(22) Date of filing: 19.07.2024
(51) Int. Cl.: G16B 20/50, G16B 40/20, G16B 30/00, G06N 3/042

(54) **PREDICTING STABILITY OF PROTEIN ON THE BASIS OF GRAPH NEURAL NETWORK**

(30) Priority: 21.07.2023 CN 202310905338
(71) Applicant: Genscript (Shanghai) Biotech Co., Ltd., Shanghai 200131 (CN)
(72) Inventor: LI, Gen, Shanghai 200131 (CN); FAN, Long, Shanghai 200131 (CN); YAO, Sijie, Shanghai 200131 (CN)
(74) Representative: Synergy IP Group AG
(86) International application number: PCT/CN2024/106390
(87) International publication number: WO 2025/021024

(57) **Abstract**

A method for predicting stability of protein on the basis of a graph neural network is provided, in particular, a method for predicting stability of protein after mutation is provided. The method comprises: on the basis of sequence information of a protein sample to be predicted, using a pre-trained model to perform feature extraction, so as to obtain a sequence feature of said protein sample; on the basis of three-dimensional structure information of said protein sample, obtaining a structural feature of said protein sample; and, on the basis of the sequence feature and the structural feature, using a graph neural network based prediction model to predict, so as to determine a stability prediction result of said protein sample after mutation. Further provided is a method for constructing a stability prediction model for protein after mutation. Combining a natural language processing model with a graph neural network for predicting stability changes caused by mutation presents a performance superior to that of earlier methods.

## Description

### CROSS-REFERENCE TO RELATED APPLICATIONS

The present application claims priority to the Chinese Patent Application No. 202310905338.0 entitled "PREDICTING STABILITY OF PROTEIN ON THE BASIS OF GRAPH NEURAL NETWORK" and filed with the China National Intellectual Property Administration on July 21, 2023, which is incorporated herein by reference in its entirety.

### TECHNICAL FIELD

The present disclosure relates to the study of protein stability, and in particular, relates to predicting protein stability on the basis of a graph neural network (GNN).

### BACKGROUND

Proteins can perform specific functions only by adopting a particular three-dimensional structure, and their ability to fold into corresponding three-dimensional structures is influenced by protein stability. (See Ittisoponpisan, Sirawit et al., 2019, "Can Predicted Protein 3D Structures Provide Reliable Insights into Whether Missense Variants Are Disease Associated?", Journal of Molecular Biology 431(11): 2197-2212 and Ofoegbu, Tochukwu C. et al., 2019, "PhyreRisk: A Dynamic Web Application to Bridge Genomics, Proteomics and 3D Structural Data to Guide Interpretation of Human Genetic Variants", Journal of Molecular Biology 431(13): 2460-66, which are incorporated herein by reference in their entireties and made a part of the present disclosure.) In many fields ranging from biotechnology to medicine, accurately predicting the impact of mutations on protein stability (folding free energy) is of critical importance. For example, in protein engineering, the targeted redesign of proteins makes the optimization of the biotechnology and biopharmaceutical processes involved with these proteins become possible. (See Coluzza, Ivan, 2017, "Computational Protein Design: A Review", Journal of Physics: Condensed Matter 29(14): 143001 and Korendovych, Ivan V. and William F. DeGrado, 2020, "De Novo Protein Design, A Retrospective", Quarterly Reviews of Biophysics 53: e3, which are incorporated herein by reference in their entireties and made a part of the present disclosure.) Stability prediction also plays a crucial role in interpreting the impact of human genetic variations, helping to better understand how these variations contribute to diseases. (See Gunning, Adam C et al., 2021, "Assessing Performance of Pathogenicity Predictors Using Clinically Relevant Variant Datasets", Journal of Medical Genetics 58(8): 547-55 and Kopanos, Christos et al., 2019, "VarSome: The Human Genomic Variant Search Engine", Jonathan Wren ed., Bioinformatics 35(11): 1978-80, which are incorporated herein by reference in their entireties and made a part of the present disclosure.) Directed evolution and rational design are two common strategies for protein modification. The directed evolution strategy has been successfully applied to the modification of enzyme properties such as enzyme activity, stability, substrate specificity, and stereoselectivity. For this achievement, American scientist Frances H. Arnold was awarded the 2018 Nobel Prize in Chemistry. However, directed evolution requires the construction of a large-scale mutant library and the establishment of high-throughput screening methods, which consume substantial human, material, and financial resources. The directed evolution method also finds it difficult to achieve a comprehensive search of sequence space, and thus has inherent flaws. The rational design strategy relies on understanding of the relationship between the structure and function of a protein to predict potential mutants, and then constructs these mutants in target genes through site-directed mutagenesis. Essentially, compared to directed evolution, rational design offers higher efficiency in protein modification. Additionally, the rational design method is universal, and an effective rational design strategy can be universally applied to the modification of various proteins. However, currently the accuracy of the rational design methods is generally low, and their application scope is far less extensive than that of directed evolution. Over the past few decades, numerous studies have focused on developing newer and more effective methods, with predicting the impact of mutations on protein stability on the basis of artificial intelligence being the latest research and development direction. In recent years, artificial intelligence-aided protein engineering has gradually evolved into an efficient new strategy for protein design. It has demonstrated unique advantages in multiple aspects such as protein structure prediction, function prediction, stability prediction, and antibody affinity prediction, emerging as a technological wave again following rational design and directed evolution.

Over the past decade, artificial intelligence has brought unprecedented improvements to various protein prediction methods, ranging from predicting residue interactions on the basis of protein sequence embedding layers to predicting protein structures and functions. Among the various fields of artificial intelligence, natural language processing is currently one of the most popular. It is often used for text generation, question answering, and translation. Natural language processing is inherently applicable to analysing the structure and meaning of texts. Meanwhile, the most fundamental nucleic acids and proteins in life are both composed of sequences, which have an inherent connection with texts. The core concept behind these methods is to interpret protein sequences into sentences, and their constituent parts - amino acids as individual words. These methods have demonstrated the ability to directly extract specific features from the amino acid sequences of proteins, overcoming the limitations of standard feature-based machine learning (ML) methods. A variety of protein-based natural language models have been developed to characterize the features of protein sequences, which are then applied to downstream protein property prediction and have demonstrated excellent performance. (See Rao, Roshan et al., 2019, "Evaluating Protein Transfer Learning with TAPE", Advances in Neural Information Processing Systems 32: 9689-9701, which is incorporated herein by reference in its entirety and made a part of the present disclosure.)

By contrast, geometric deep learning methods, particularly graph neural networks (GNNs), can predict various properties of proteins through convolution operations on protein structures. If the three-dimensional structure of a protein is viewed as a network where each amino acid is directly connected according to certain relationships, then the connections between each node and other nodes represent the interactions formed between a certain amino acid and other surrounding amino acids. These interactions precisely reflect the characteristics of the protein structure. This feature is generally referred to as an edge feature of a graph, in which the weight of an edge can be represented by the distance between nodes. For example, in the structure of a pharmaceutical molecule, the weight of the edge between two atoms can be determined by the distance therebetween or the type of a chemical bond. In consideration of edge features and weights, GNNs can more accurately capture the relationships and distances between nodes, thereby improving the expressive ability and performance of the model. GNNs have achieved tremendous success across various domains, ranging from learning features for a quantitative structure-activity relationship model, to predicting the biochemical activity of medicine, and to predicting an interface between protein pairs.

### SUMMARY

On the basis of the problems existing in the current methods for predicting the impact of mutations on protein stability as mentioned above, the present disclosure proposes a fast and accurate method for predicting the impact of mutations on protein stability. This is a method based on the latest natural language processing techniques, in which a T5 (i.e., Transfer Text-to-Text Transformer) pre-trained model is used to extract sequence features. The features are combined with structure-based features and input into a graph neural network model, preferably a graph convolutional network model, to predict changes in folding free energy caused by amino acid substitutions, thereby obtaining the prediction results of protein stability. The results demonstrate further performance improvement compared to existing methods.

According to a first aspect of the present disclosure, provided is a method for predicting stability of a protein after mutation. The method may include the following steps: performing feature extraction using a pre-trained model on the basis of sequence information of a protein sample to be predicted, so as to obtain a sequence feature of the protein sample to be predicted; obtaining a structural feature of the protein sample to be predicted on the basis of three-dimensional structural information of the protein sample to be predicted; and performing prediction using a graph neural network-based prediction model on the basis of the sequence feature and the structural feature, so as to determine a prediction result for stability of the protein sample to be predicted after mutation.

In the method according to the first aspect of the present disclosure, the sequence information of the protein sample to be predicted may include amino acid sequence information of the protein sample to be predicted before mutation and after mutation.

In another aspect, the sequence information of the protein sample to be predicted may include amino acid sequence information of the protein sample to be predicted before mutation and specified mutation information. Thus, amino acid sequence information of the protein sample to be predicted after mutation can be obtained on the basis of the amino acid sequence information of the protein sample to be predicted before mutation and the specified mutation information.

Preferably, the specified mutation information includes a specified mutation site and/or a specified amino acid type after mutation.

In the method according to the first aspect of the present disclosure, said performing feature extraction using the pre-trained model on the basis of the sequence information of the protein sample to be predicted, so as to obtain the sequence feature of the protein sample to be predicted, may further includes: performing feature extraction separately on the amino acid sequence information of the protein sample to be predicted before mutation and after mutation using the pre-trained model, so as to obtain sequence feature information before mutation and sequence feature information after mutation; and concatenating the sequence feature information before mutation and the sequence feature information after mutation, so as to obtain the sequence feature of the protein sample to be predicted.

In the method according to the first aspect of the present disclosure, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

In the method according to the first aspect of the present disclosure, the three-dimensional structural information of the protein sample to be predicted can include the three-dimensional structural information of the protein sample to be predicted obtained through at least one of the following databases or prediction software: PDB database, AlphaFold2, I-TASSER, RoseTTAFold, Modeller, and Swiss-model.

In the method according to the first aspect of the present disclosure, the three-dimensional structural information may include an interaction network, a secondary structure, an amino acid residue distance, or a physical environment. Preferably, the three-dimensional structural information includes an interaction network.

Preferably, the three-dimensional structural information of the protein sample to be predicted is acquired by: acquiring coordinates of a three-dimensional structure of the protein sample to be predicted; calculating distances between amino acid residue pairs in the three-dimensional structure of the protein on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protein sample to be predicted.

Correspondingly, in the method according to the first aspect of the present disclosure, said obtaining the structural feature of the protein sample to be predicted on the basis of the three-dimensional structural information of the protein sample to be predicted, may include: taking a mutation site as the centre, extracting an adjacency matrix of a specified size from the adjacency matrix of the entire protein sample to be predicted, which is used as the structural feature of the protein sample to be predicted; or recalculating distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between the nodes, recorded as 1, otherwise recorded as 0, with a self-connection recorded as 0, thereby obtaining a second-order adjacency matrix of the protein sample to be predicted. In some implementations, taking a mutation site as the centre, an N*N adjacency matrix (where N is an integer greater than or equal to 1) is extracted from the adjacency matrix of the entire protein. Said extracting the N*N adjacency matrix from the adjacency matrix of the entire protein can be performed on the basis of sequences on the left and right sides of the mutation site or on the basis of distances from the mutation site. In an implementation, taking a mutation site as the centre, an amino acid within a distance of less than 10 angstroms from the mutation site is selected from the adjacency matrix of the entire protein sample to be predicted, which is used as a structural feature of the protein sample to be predicted.

In the method according to the first aspect of the present disclosure, said performing prediction using the graph neural network-based prediction model on the basis of the sequence feature and the structural feature, so as to determine the prediction result for stability of the protein sample to be predicted after mutation, may further include: inputting the sequence feature and the structural feature into the graph neural network-based prediction model; updating the features through matrix multiplication and aggregation operations using several layers of graph network structures, and then outputting the updated features; merging the features output by the several layers of graph network structures, respectively; compressing the features using a pooling operation; and performing stability classification on the features on the basis of a classification function of an output layer. Preferably, the pooling operation may be average pooling, max pooling, or K-max pooling.

In the method according to the first aspect of the present disclosure, the graph neural network-based prediction model can be constructed through the steps of: acquiring a training set, where the training set includes information of a plurality of training samples, and the information of each of the training samples includes three-dimensional structural information of a sample protein, amino acid sequence information of the sample protein before mutation and after mutation, and a mutation stability label; performing feature extraction using a pre-trained model on the basis of the amino acid sequence information of the sample protein before mutation and after mutation, so as to obtain sequence features of the plurality of training samples; obtaining structural features of the plurality of training samples on the basis of the three-dimensional structural information of the sample protein; and training a graph neural network on the basis of the sequence features, the structural features, and the mutation stability labels, so as to obtain the graph neural network-based prediction model.

Preferably, the graph neural network-based prediction model is a classifier.

Preferably, the graph neural network is a graph convolutional network.

More preferably, the graph neural network-based prediction model includes one of the following models: MGC model, MGC_F model, MGC_S model, GINConv model, and SageConv model.

Preferably, experimental data from a public database can be obtained as the training set. Meanwhile, the size of the training set can be doubled by performing inversion processing on the experimental data from the public database.

According to a second aspect of the present disclosure, provided is a construction method for a prediction model for stability of a protein after mutation. The method may include: acquiring a training set, where the training set includes information of a plurality of training samples, and the information of each of the training samples includes three-dimensional structural information of a sample protein, amino acid sequence information of the sample protein before mutation and after mutation, and a mutation stability label; performing feature extraction using a pre-trained model on the basis of the amino acid sequence information of the sample protein before mutation and after mutation, so as to obtain sequence features of the plurality of training samples; obtaining structural features of the plurality of training samples on the basis of the three-dimensional structural information of the sample protein; and training a graph neural network on the basis of the sequence features, the structural features, and the mutation stability labels, so as to obtain a graph neural network-based prediction model.

In the method according to the second aspect of the present disclosure, said performing feature extraction using the pre-trained model on the basis of the amino acid sequence information of the sample protein before mutation and after mutation, so as to obtain the sequence features of the plurality of training samples, may further include: performing feature extraction separately on the amino acid sequence information of the sample protein before mutation and after mutation using the pre-trained model, so as to obtain sequence feature information before mutation and sequence feature information after mutation; and concatenating the sequence feature information before mutation and the sequence feature information after mutation, so as to obtain the sequence features of the plurality of training samples.

In the method according to the second aspect of the present disclosure, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

In the method according to the second aspect of the present disclosure, the three-dimensional structural information of the sample proteins may include three-dimensional structural information of the sample proteins obtained through at least one of the following databases or prediction software: PDB database, AlphaFold2, I-TASSER, RoseTTAFold, Modeller, and Swiss-model.

In the method according to the second aspect of the present disclosure, the three-dimensional structural information may include an interaction network, a secondary structure, an amino acid residue distance, or a physical environment. Preferably, the three-dimensional structural information includes an interaction network.

Preferably, the three-dimensional structural information of the sample protein may be acquired by: acquiring coordinates of a three-dimensional structure of the sample protein; calculating distances between amino acid residue pairs in the three-dimensional structure of the sample protein on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire sample protein.

Correspondingly, in the method according to the second aspect of the present disclosure, said obtaining the structural feature of the plurality of training samples on the basis of the three-dimensional structural information of the protein sample, includes: taking a mutation site as the centre, extracting an adjacency matrix of a specified size from the adjacency matrix of the entire sample protein, which is used as the structural feature of the sample protein; or recalculating distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between the nodes, recorded as 1, otherwise recorded as 0, with a self-connection recorded as 0, thereby obtaining a second-order adjacency matrix of the protein sample to be predicted.

In the method according to the second aspect of the present disclosure, said training the graph neural network on the basis of the sequence features, the structural features, and the mutation stability labels, so as to obtain the graph neural network-based prediction model, may include: inputting the sequence features and the structural features into the graph neural network; updating the features through matrix multiplication and aggregation operations using several layers of graph network structures, and then outputting the updated features; merging the features output by the several layers of graph network structures, respectively; compressing the features using a pooling operation; performing stability classification on the features on the basis of a classification function of an output layer; and taking the mutation stability labels as an output target, adjusting network parameters of the graph neural network. Preferably, the pooling operation may be average pooling, max pooling, or K-max pooling.

Preferably, the graph neural network-based prediction model is a classifier.

Preferably, the graph neural network is a graph convolutional network.

More preferably, the graph neural network-based prediction model includes one of the following models: MGC model, MGC_F model, MGC_S model, GINConv model, and SageConv model.

Preferably, experimental data from a public database can be obtained as the training set. Meanwhile, the size of the training set can be doubled by performing inversion processing on the experimental data from the public database.

According to a third aspect of the present disclosure, provided is a system for predicting stability of a protein after mutation. The system may include: an acquisition module, configured to acquire sequence information and three-dimensional structural information of a protein sample to be predicted; and a processing module, configured to obtain a prediction result for stability of the protein sample to be predicted after mutation by inputting the sequence information and the three-dimensional structural information of the protein sample to be predicted. The processing module further includes the following sub-modules: a sequence feature sub-module, configured to perform feature extraction using a pre-trained model on the basis of the sequence information of the protein sample to be predicted, so as to obtain a sequence feature of the protein sample to be predicted; a structural feature sub-module, configured to obtain a structural feature of the protein sample to be predicted on the basis of the three-dimensional structural information of the protein sample to be predicted; and a model prediction sub-module, configured to perform prediction using a graph neural network-based prediction model on the basis of the sequence feature and the structural feature, so as to determine a prediction result for stability of the protein sample to be predicted after mutation.

The system for predicting the protein stability on the basis of the graph neural network according to the third aspect of the present disclosure can be implemented by a computer. Preferably, the system can implement the following operations by executing a computer program: acquiring sequence information and three-dimensional structural information of a protein sample to be predicted; and obtaining a prediction result for stability of the protein sample to be predicted after mutation by inputting the sequence information and the three-dimensional structural information of the protein sample to be predicted, where the operation further includes the following sub-operations: performing feature extraction using a pre-trained model on the basis of the sequence information of the protein sample to be predicted, so as to obtain a sequence feature of the protein sample to be predicted; obtaining a structural feature of the protein sample to be predicted on the basis of the three-dimensional structural information of the protein sample to be predicted; and performing prediction using a graph neural network-based prediction model on the basis of the sequence feature and the structural feature, so as to determine a prediction result for stability of the protein sample to be predicted after mutation.

According to a fourth aspect of the present disclosure, provided is a non-transitory computer-readable storage medium for storing a computer program. The computer program includes instructions. The instructions, when executed by a processor of an electronic device, cause the electronic device to perform the method for predicting stability of the protein after mutation according to the first aspect of the present disclosure, or the construction method for the prediction model for stability of the protein after mutation according to the second aspect of the present disclosure.

According to a fifth aspect of the present disclosure, provided is a computer system. The computer system includes: a processor, a memory, and a computer program. The computer program is stored in the memory and configured to be executed by the processor. The computer program includes instructions for performing the method for predicting stability of the protein after mutation according to the first aspect of the present disclosure, or the construction method for the prediction model for stability of the protein after mutation according to the second aspect of the present disclosure.

The above methods are based on an advanced natural language processing model combined with a graph neural network, can be applied to various biological fields, and exhibit superior performance to earlier methods.

### BRIEF DESCRIPTION OF THE DRAWINGS

The present disclosure will be more fully understood from the following detailed description in conjunction with the accompanying drawings, in which similar elements are numbered in a similar manner:
FIG. 1 is a flow chart of a method for predicting stability of a protein after mutation according to the embodiments of the present disclosure.
FIG. 2 is a joint schematic diagram illustrating a method for predicting stability of a protein after mutation and a construction method for a prediction model for stability of a protein after mutation according to the embodiments of the present disclosure.
FIG. 3 is a flow chart of a construction method for a prediction model for stability of a protein after mutation as used in the present disclosure.
FIG. 4 is a schematic diagram of an adjacency matrix.
FIG. 5 is a schematic diagram illustrating the formation of a node feature matrix.
FIG. 6 is a schematic diagram illustrating the structure-based construction of an adjacency matrix.
FIG. 7 is a schematic diagram illustrating the sequence-based construction of a node feature matrix.
FIG. 8 is a schematic diagram of the framework of the prediction model.
FIG. 9 is a schematic diagram integrating FIGs. 6-8.
FIG. 10 shows a schematic block diagram of a system for predicting stability of a protein after mutation according to the present disclosure.

### DETAILED DESCRIPTION

Unless otherwise stated, technical and scientific terms used herein have the same meaning as that commonly understood by those of ordinary skill in the art to which the present disclosure pertains.

The technical solutions of the present disclose are further described below through embodiments and in conjunction with the accompanying drawings. Unless otherwise stated, the methods and materials of the embodiments described below are all conventional products commercially available. It will be understood by those skilled in the art to which the present disclosure pertains that the methods and materials described below are merely exemplary, and should not be construed as limiting the scope of the present disclosure.

Current methods for predicting the impact of mutations on protein stability are mainly classified into structure-based and sequence-based methods. The structure-based methods generally exhibit slightly superior performance to sequence-based ones, whereas the sequence-based methods are not limited by the lack of structures. Although only approximately 0.2% of proteins in the UniProt database have available three-dimensional structures (see Quan, Lijun, Qiang Lv, and Yang Zhang, 2016, "STRUM: Structure-Based Prediction of Protein Stability Changes upon Single-Point Mutation," Bioinformatics 32(19): 2936-46, which is incorporated herein by reference in its entirety and made a part of the present disclosure), the emergence of software tools such as AlphaFold2 has greatly mitigated the limitations on the application of structure-based methods caused by the lack of three-dimensional structures.

At present, input features generated by natural language models that contain only sequence information can already achieve excellent performance (for example, see Chinese Patent Application No. CN202211708226.8, which is incorporated herein by reference in its entirety and made a part of the present disclosure). Similarly, using deep neural networks to extract structural features from the three-dimensional structure of proteins can also yield good performance. However, significant challenges remain in achieving further improvements. Therefore, introducing graph neural networks into protein stability prediction and combining them with protein natural language models would be a promising method.

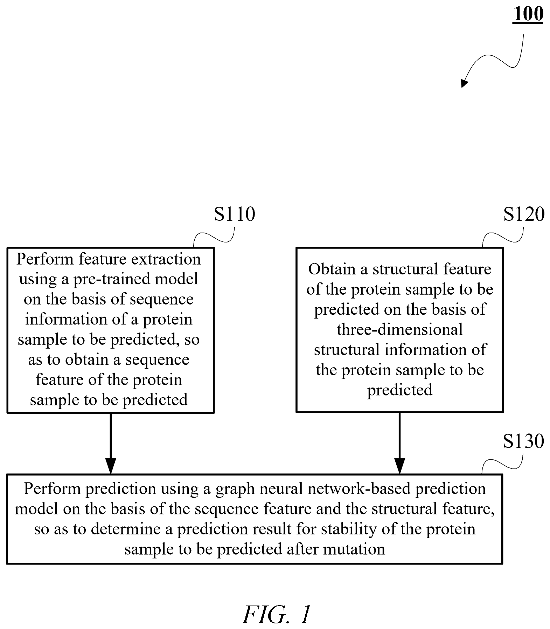

From the overall perspective of prediction methods, FIG. 1 shows a flow chart of a method for predicting stability of a protein after mutation according to the embodiments of the present disclosure.

As shown in FIG. 1, the method 100 for predicting stability of the protein after mutation according to the embodiments of the present disclosure starts at step S110. In this step, feature extraction is performed using a pre-trained model on the basis of sequence information of a protein sample to be predicted, so as to obtain a sequence feature of the protein sample to be predicted.

The sequence information of the protein sample to be predicted described here may include amino acid sequence information of the protein sample to be predicted before mutation and after mutation.

In another case, the sequence information of the protein sample to be predicted may also include amino acid sequence information of the protein sample to be predicted before mutation and specified mutation information. In this case, amino acid sequence information of the protein sample to be predicted after mutation can be obtained on the basis of the amino acid sequence information of the protein sample to be predicted before mutation and the specified mutation information. The specified mutation information may include a specified mutation site and/or a specified amino acid type after mutation. For example, in the case that the specified mutation information is a specified mutation site, the amino acid at this site can be altered to form the amino acid sequence information of the protein sample to be predicted after mutation. In the case that the specified mutation information further includes a specified amino acid type after mutation, the amino acid sequence information after mutation may be a single amino acid sequence. In the case that the specified mutation information does not include a specified amino acid type after mutation, the amino acid sequence information after mutation may consist of 19 amino acid sequences, in which the amino acid at the mutation site is replaced with each of the other 19 types of amino acids. In another case that the specified mutation information only includes a specified amino acid type after mutation, it is necessary to sequentially alter the amino acid at each site in the sequence before mutation, thereby forming N sequences after mutation (where N is the number of sites in the sequence). In addition, in another extreme case that the specified mutation sites are multiple sites or even all sites, or the specified amino acid types after mutation are multiple types or all the other 19 types, it is necessary to traverse all possible mutations to form up to 19*N sequences after mutation.

It should be understood by those skilled in the art that in subsequent steps, it is necessary to perform feature extraction separately on sequences before mutation and after mutation, which are then concatenated to form sequence-related features. Therefore, regardless of the initially acquired protein sequence information, it is necessary to form sequences before mutation and after mutation when executing step S110.

Specifically, in step S110, feature extraction is performed separately on amino acid sequence information of a protein sample to be predicted before mutation and after mutation using a pre-trained model, so as to obtain sequence feature information before mutation and sequence feature information after mutation. Subsequently, still in step S110, the sequence feature information before mutation and the sequence feature information after mutation are concatenated, so as to obtain the sequence feature of the protein sample to be predicted.

In the research area of natural language processing, with the continuous enhancement of computer computing power, an increasing number of pre-trained models (PTMs) for general language representation have gradually emerged. This is of great help to a downstream preprocessing task, as it enables the learning of knowledge about language itself from massive unlabeled data, followed by fine-tuning on a small amount of labeled data, thereby allowing the downstream task to better learn both the inherent features of the language itself and the knowledge of a specific task. By training on a protein or nucleic acid sequence corpus, a pre-trained model applicable to the field of biology can be obtained. Existing models trained on the protein sequence corpus mainly include ESM-1b, UniRef, ProteinBert, TAPE, ProtGPT2, ProtTXL, ProtBert, ProtXLNet, ProtAlbert, ProtElectra, ProtT5-XL, and ProtT5-XXL. Although the pre-trained model can also be obtained by training on the protein sequence corpus by ourselves, the performance of such self-trained models generally fails to reach the level of the above models due to constraints in cost and time.

As described above, the pre-trained model is a natural language model in nature. In the field of biology, a pre-trained model that has been improved and optimised according to the characteristics and purposes of the field of biology is generally used. Such pre-trained model has already learned knowledge related to the field of biology from a large amount of unlabeled sequence data in an existing protein corpus, and can be used to extract features related to mutation from the protein sequence information of training samples. Furthermore, by training on the basis of the extracted features related to mutation, model parameters can be optimised using only a small number of labeled training samples. For example, the pre-trained model described in the present disclosure may be implemented using one or a combination of more of the models mentioned above and listed again below: ESM-1b, UniRef, ProteinBert, TAPE, ProtGPT2, ProtTXL, ProtBert, ProtXLNet, ProtAlbert, ProtElectra, ProtT5-XL, and ProtT5-XXL. Preferably, the pre-trained model may be ProtT5-XL or ProtT5-XXL. More preferably, the pre-trained model is ProtT5-XL. That is, the pre-trained model used here is preferably a T5 (i.e., Transfer Text-to-Text Transformer) pre-trained model based on the latest natural language processing method.

The purpose of step S110 is to obtain a sequence feature of the protein sample to be predicted. The purpose of step S120, which will be described next, is to obtain a structural feature of the protein sample to be predicted.

In step S120, a structural feature of the protein sample to be predicted is obtained on the basis of three-dimensional structural information of the protein sample to be predicted.

In addition to obtaining related features through a pre-trained model, related features can also be obtained in combination with three-dimensional structural information of a protein for information supplementation. The three-dimensional structural information can be obtained from the PDB database (https://www.rcsb.org/) or by means of prediction software, such as the following (with reference descriptions or sources in parentheses):
AlphaFold2 (https://alphafold.ebi.ac.uk/);
I-TASSER (https://zhanggroup.org/I-TASSER/);
RoseTTAFold (see Minkyung Baek et al., "Accurate Prediction of Protein Structures and Interactions Using a Three-track Neural Network", Science 373, 871-876 (2021). DOI:10.1126/science.abj8754);
Modeller (https://salilab.org/modeller/);
Swiss-model (https://swissmodel.expasy.org/), etc.

The obtainable three-dimensional structural information of a protein usually includes a secondary structure, an interaction network, an amino acid residue distance, a physical environment, energy information derived from the three-dimensional structure, etc. Among them, the interaction network is one of the most commonly used features in structure-based prediction methods, as it contains contact information between all residue pairs.

According to preferred embodiments of the present disclosure, the three-dimensional structural information used is an interaction network. More specifically, a GNN, more specifically a graph convolutional network (GCN) prediction model, is selected based on features of the interaction network.

It should be understood by those skilled in the art that the present disclosure does not limit the sequential order of step S110 and step S120. That is, the sequence feature and the structural feature can be obtained in any order, or simultaneously.

Finally, in step S130, prediction is performed using a graph neural network-based prediction model on the basis of the sequence feature obtained in step S110 and the structural feature obtained in step S120, so as to determine a prediction result for stability of the protein sample to be predicted after mutation.

The graph neural network-based prediction model is actually a classifier.

In step S130, the sequence-based feature and the structure-based feature are input into the classifier to perform prediction for a specific task. Classifiers are generally classified into machine learning methods and deep learning methods. Commonly used machine learning methods include: linear regression algorithm, support vector machine (SVM) algorithm, K-nearest neighbours (KNN) algorithm, logistic regression (LR) algorithm, decision tree algorithm, K-means algorithm, random forest algorithm, naive Bayes algorithm, gradient boosting algorithm, and ensemble learning method. Deep learning methods generally include: convolutional neural network, recurrent neural network, recursive neural network, and long short-term memory (LSTM) network. A graph neural network (GNN) is a deep learning model based on graph-structured data, which is used to process structured data involving nodes and edges. A graph convolutional network (GCN) is a GNN model based on the convolutional neural network (CNN), which realises effective representation and learning of node features by aggregating information of neighbouring nodes. A graph attention network (GAT) is a GNN model based on the attention mechanism, which realises adaptive aggregation of node features by learning the attention weights of neighbouring nodes, and can better handle the relationships between different nodes. GraphSAGE (Graph Sampling and Aggregation, SageConv) is a GNN model based on sampling, which realises effective learning and representation of node features by sampling and aggregating neighbouring nodes. GraphSAGE can handle large-scale graph data while ensuring the efficiency and accuracy of the model. A graph isomorphism network (GINConv) is a GNN model based on graph isomorphism, which realises effective learning and representation of node features by aggregating features of neighbouring nodes and combining the aggregated result with the features of the node itself. A diffusion convolutional neural network (DCNN) is a GNN model based on a diffusion process, which realises effective learning and representation of node features by diffusing and aggregating node features. Multi-graph convolution (MGC) can perform graph convolution on different types of graphs, merge them together, and make full use of information from different types of graphs. Among them, MGC-F indicates that the input adjacency matrix is a first-order adjacency matrix, while MGC-S indicates that the input adjacency matrix is a second-order adjacency matrix. These methods all have their own advantages and application scopes, and appropriate methods can be selected on the basis of specific tasks and data characteristics.

In a preferred embodiment of the present disclosure, the graph neural network is a graph convolutional network.

In more preferred embodiments of the present disclosure, the graph neural network-based prediction model includes one of the following models: MGC model, MGC_F model, MGC_S model, GINConv model, and SageConv model. For more details, reference can be made to the detailed description below.

With regard to the construction method for the prediction model, reference can be made to the detailed description below.

As described above, the methods for predicting the impact of mutation on stability of a protein in the prior art are mainly classified into structure-based and sequence-based methods. Input features generated by natural language models that contain only sequence information can already achieve excellent performance. Similarly, using a deep neural network to extract structural features from the three-dimensional structure of a protein can also yield good performance.

The technique provided in the present disclosure differs from the above prior art in that the technique of the present disclosure introduces a graph neural network, more specifically a graph convolutional network, into the prediction of stability of a protein after mutation, which is combined with a mature protein natural language model (pre-trained model) in the prior art to jointly contribute to the determination of a prediction result. In other words, the sequence features and structural features of the protein sample to be predicted are combined to jointly serve as the input for the graph neural network-based prediction model, thereby outputting a prediction result regarding stability of the protein after mutation. The prediction result determined as such is superior to that determined by the methods of the prior art.

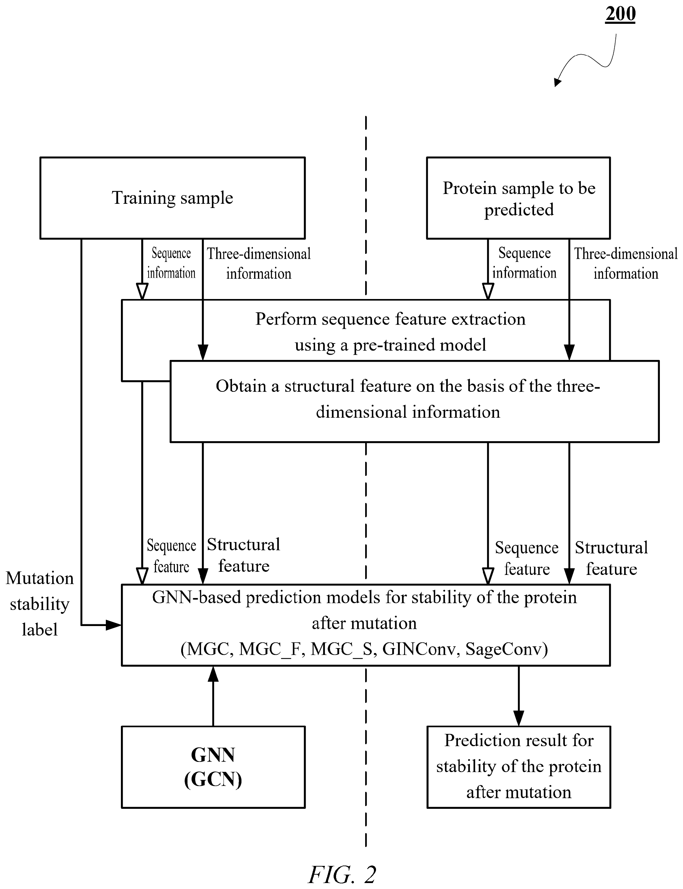

FIG. 2 presents a joint schematic diagram 200 illustrating a method for predicting stability of a protein after mutation and a construction method for a prediction model for stability of a protein after mutation according to the embodiments of the present disclosure. In the schematic diagram 200 shown in FIG. 2, the left side of the dashed line shows a construction method for a model, while the right side of the dashed line shows a method of using the model to perform prediction.

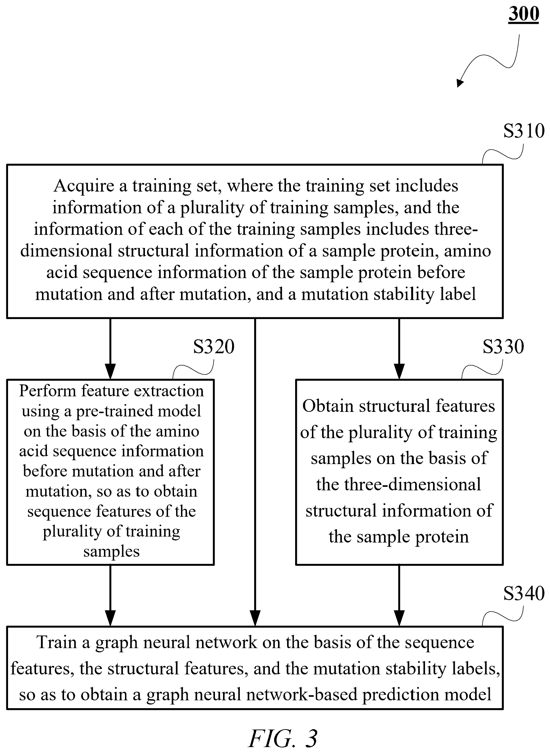

The flow on the right side of the dashed line in FIG. 2 is actually the method for predicting stability of the protein after mutation according to the embodiments of the present disclosure as shown in FIG. 1. The flow on the left side of the dashed line in FIG. 2 will be described in detail below (e.g., FIG. 3 and corresponding textual description thereof). As can be seen in FIG. 2, both the model construction and the actual prediction process require the use of two feature extraction steps, namely: sequence feature extraction and structural feature extraction. For the training sample, the information of the training sample includes three-dimensional structural information of the sample protein, amino acid sequence information of the sample protein before mutation and after mutation, and a mutation stability label. As described below, the mutation stability labels can be categorized into destabilizing mutations and stabilizing mutations. Destabilizing mutations include those with decreased stability or loss of stability, while stabilizing mutations include those with increased stability or unchanged stability. Therefore, in the process of model construction, sequence information from training samples is used to extract sequence features. Structural features are extracted through three-dimensional structural information. The GNN-based prediction model, i.e., the classifier, is fully trained using sequence features and structural features as input data, and mutation stability labels from training samples as output data, thereby obtaining the final GNN-based model for predicting stability of the protein after mutation. The constructed prediction model can then be put into practical use for stability prediction of the protein after mutation, namely the flow on the right side of the dashed line in FIG. 2. In addition, it should be noted that in FIG. 2, hollow arrows are used to indicate the signal transmission process for sequence information and sequence features; while solid arrows distinct from the hollow arrows are used to represent the signal transmission process for three-dimensional structural information and structural features. In addition, all other signal flows and control flows are also denoted using solid arrows.

The flow of the construction method for the prediction model for stability of protein after mutation according to the embodiments of the present disclosure is described in detail below.

FIG. 3 is a flow chart of a construction method 300 for a prediction model for stability of protein after mutation as used in the present disclosure.

It is known to those skilled in the art that as shown in FIG. 3, in step S310, a training set should first be obtained, where the training set includes information of a plurality of training samples. Specifically, the information of each of the training samples includes three-dimensional structural information of the sample protein, amino acid sequence information of the sample protein before mutation and after mutation, and a mutation stability label.

Artificial intelligence (AI) algorithms rely heavily on data. The quantity and quality of initial data determine the generalization performance of the trained model, which is most directly reflected in the prediction accuracy of the model. Insufficient sample size or low quality in a dataset will lead to overfitting or underfitting in the model. To solve this problem, the most effective method is to acquire more experimental data as the training set. The main source for acquiring experimental data currently is public databases. For the task of predicting the impact of mutations on proteins using sequence-based methods, the dataset size can be doubled by applying inversion processing to the data in the databases. "Inversion" means that under the same environmental conditions, the change in folding free energy (ΔΔG) exhibits reverse symmetry in terms of free energy during mutation. More accurately, if protein B is a mutant of protein A, then ΔΔG (A → B) = -ΔΔG (B → A), which is consistent with the law of energy conservation in physics. According to the above definition, merging the original data with the inverted data can double the original data volume. By using such an inversion method, the original dataset is not only expanded, but also balanced, which enhances the robustness of the final prediction model.

It should be noted that this method of expanding the dataset by inverting the original dataset is performed only for the training set.

In the specific embodiments below, the acquisition of the training dataset will be described in more detail.

Mutation stability labels can be used to reflect the trend of how amino acid mutations affect the thermal stability of proteins. In some embodiments, mutation stability labels can be determined based on the change in folding free energy (ΔΔG) of protein mutants. The change in folding free energy (ΔΔG) caused by mutations is closely related to the thermal stability of the mutant. ΔΔG = folding free energy of the protein before mutation - folding free energy of the protein after mutation. A ΔΔG value less than 0 indicates that the stability of the protein is enhanced after mutation, while a ΔΔG value greater than 0 indicates that the mutation reduces the stability of the protein. Illustratively, the mutation stability labels can be categorized into destabilizing mutations and stabilizing mutations. Destabilizing mutations include those with decreased stability or loss of stability, while stabilizing mutations include those with increased stability or unchanged stability. It can be understood that using mutation stability information as a label for a training sample enables the prediction model obtained through training to output a prediction result related to the impact of an amino acid mutation on the thermal stability of the protein.

In step S320, feature extraction is performed using a pre-trained model on the basis of amino acid sequence information of the sample protein before mutation and after mutation, so as to obtain sequence features of the plurality of training samples.

Specifically, in step S320, feature extraction is performed separately on the amino acid sequence information of the sample protein before mutation and after mutation using the pre-trained model, so as to obtain sequence feature information before mutation and sequence feature information after mutation. Subsequently, still in step S320, the sequence feature information before mutation and the sequence feature information after mutation are concatenated, so as to obtain the sequence features of the plurality of training samples.

As described above, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL. Preferably, the pre-trained model is ProtT5-XL or ProtT5-XXL. More preferably, the pre-trained model is ProtT5-XL. That is, the pre-trained model used here is preferably a T5 (i.e., Transfer Text-to-Text Transformer) pre-trained model based on the latest natural language processing method.

The purpose of step S320 is to obtain the sequence feature of the training sample. The purpose of step S330, which will be described next, is to obtain the structural feature of the training sample.

In step S330, structural features of the plurality of training samples are obtained on the basis of the three-dimensional structural information of the sample protein.

As described above, the three-dimensional structural information of the sample protein can be obtained through at least one of the following databases or prediction software: PDB database, AlphaFold2, I-TASSER, RoseTTAFold, Modeller, Swiss-model, etc.

The obtainable three-dimensional structural information of a protein usually includes a secondary structure, an interaction network, an amino acid residue distance, a physical environment, energy information derived from the three-dimensional structure, etc. Among them, the interaction network is one of the most commonly used features in structure-based prediction methods, as it contains contact information between all residue pairs.

According to preferred embodiments of the present disclosure, the three-dimensional structural information used here is the interaction network. More specifically, a GNN, more specifically a graph convolutional network (GCN) prediction model, is selected based on features of the interaction network.

It should be understood by those skilled in the art that the present disclosure does not limit the sequential order of step S320 and step S330. That is, the sequence feature and the structural feature can be obtained in any order, or simultaneously.

In step S340, a graph neural network is trained on the basis of the sequence features, the structural features, and the mutation stability labels, so as to obtain a graph neural network-based prediction model.

As described above, the graph neural network-based prediction model is actually a classifier.

In a preferred embodiment of the present disclosure, the graph neural network is a graph convolutional network (GCN).

In more preferred embodiments of the present disclosure, the graph neural network-based prediction model includes one of the following models: MGC model, MGC_F model, MGC_S model, GINConv model, and SageConv model. For more details, reference can be made to the detailed description below.

Regarding the construction method for the prediction model, see the detailed description in the examples below.

In addition, it should be noted that: For sequence features, the pre-trained model is transferred to a new task through transfer learning by inputting the processed protein stability change dataset into the pre-trained model. In transfer learning, there are two commonly used application methods: feature extraction and fine-tuning. In feature extraction, a simple classifier can be modified or added after pre-training a network structure. The pre-trained network for the original task is used as a feature extractor for another target task, where only parameters of the newly added classifier are re-learned, while parameters of the pre-trained network remain unmodified or frozen. In the preferred embodiments of the present disclosure, a transfer learning method based on feature extraction is used.

In the process of model training, sequence features and structural features are used as input data for the GNN-based prediction model, and mutation stability labels are used as output targets of the prediction model to fully train the model. As the model parameters are continuously optimised, the output data of the prediction model will be consistent with the prediction results represented by the mutation stability labels.

More specifically, in some embodiments of the present disclosure, supervised training can be performed on the prediction model (classifier) on the basis of the sequence features, structural features, and mutation stability labels of a plurality of training samples to obtain a trained classifier. Supervised training is a process of adjusting parameters of a classifier using a set of samples with known categories to achieve the required performance. In some embodiments, a loss function (such as a cross-entropy loss function) can be constructed. The loss function is used to reflect the difference between the prediction results of the stability after mutation predicted by the classifier and the actual mutation stability labels of the training samples. Then, on the basis of this difference, the parameters of the classifier are adjusted to obtain the optimised model parameters of the classifier. When the loss function satisfies predetermined conditions, such as convergence of the loss function or the loss function value being less than a preset value, the training of the classifier is completed. In some embodiments, parameter search algorithms such as grid search and Bayesian search can be used to adjust the parameters of the classifier. In some embodiments, supervised training is a process of training the prediction model on the basis of stability labels.

It should be understood by those skilled in the art that the prediction model obtained, for example, by the method shown in FIG. 3 can be applied to the prediction method shown in FIG. 1.

Specific exemplary embodiments will be provided below.

### Embodiment methods and materials

### 1. Training dataset

To overcome the problem of insufficient samples in datasets used in previous methods, 5917 experimental samples containing thermal stability changes were reselected from recently published experimental databases, including MPTherm (https://www.iitm.ac.in/bioinfo/mptherm/) (see Kulandaisamy, A., R. Sakthivel and M. Michael Gromiha, 2021, "MPTherm: Database for Membrane Protein Thermodynamics for Understanding Folding and Stability", Briefings in Bioinformatics 22(2): 2119-25, which are incorporated herein by reference in their entireties and made a part of the present disclosure), ProThermDB (https://web.iitm.ac.in/bioinfo2/prothermdb/index.html) (see Nikam, Rahul et al., 2021, "ProThermDB: Thermodynamic Database for Proteins and Mutants Revisited after 15 Years", Nucleic Acids Research 49 (D1): D420-24, which is incorporated herein by reference in its entirety and made a part of the present disclosure), ThermoMutDB (http://biosig.unimelb.edu.au/thermomutdb/) (see Xavier, Joicymara S et al., 2021, "ThermoMutDB: A Thermodynamic Database for Missense Mutations", Nucleic Acids Research 49(D1): D475-79, which is incorporated herein by reference in its entirety and made a part of the present disclosure), and FireProtDB (https://loschmidt.chemi.muni.cz/fireprotdb/) (Stourac, Jan et al., 2021, "FireProtDB: Database of Manually Curated Protein Stability Data", Nucleic Acids Research 49(D1): D319-24, which is incorporated herein by reference in its entirety and made a part of the present disclosure). The samples include 1451 samples of stabilizing mutations and 4466 samples of destabilizing mutations. The specific screening criteria are as follows:
1) All samples that do not contain the difference in folding free energy (ΔΔG) between wild-type and mutant proteins are removed.
2) All samples with mutation information indicating non-single-point mutations are removed.
3) Duplicate samples are removed.
4) Data are inverted and merged.

The dataset used for training the model contains 11,834 samples.

### 2. Test dataset

### 2.1 Tm141

The test data is derived from the same source as the training set. The specific screening criteria are as follows:
1) All samples that do not contain the change in experimental Tm (ΔTm) before and after mutation are removed.
2) All samples with mutation information indicating non-single-point mutations are removed.
3) Duplicate samples are removed.
4) |Δtm| is greater than or equal to 15°C. The dataset used for testing the model contains 141 pieces of experimental data. It should be noted that no duplicate samples exist between the test dataset and the training set.

### 2.2 S669

To rigorously test the differences between the method of the present disclosure and other methods, a test dataset S669 was introduced, which contained 669 single-point mutation data points. When testing the dataset, duplicate data were removed from the training set. The test dataset is widely used in the following literature: Pancotti, Corrado et al., 2022, "Predicting Protein Stability Changes upon Single-Point Mutation: A Thorough Comparison of the Available Tools on a New Dataset", Briefings in Bioinformatics 23(2): bbab555, which is incorporated herein by reference in its entirety and made a part of the present disclosure.

### 3. Pre-trained model

The ProtT5-XL (ProtT5-XL-U50/ProtT5-XL-UniRef50) model used in this method was released by Ahmed Elnaggar et al. in 2021. This is a pre-trained protein model obtained by first training a T5 model on the BFD dataset and then fine-tuning the model on the UniRef50 dataset for optimization. This model contains 24 attention layers, with each layer consisting of 32 self-attention heads and 1024 neurons. See Elnaggar, Ahmed et al., 2021, "ProtTrans: Towards Cracking the Language of Lifes Code Through Self-Supervised Deep Learning and High Performance Computing", IEEE Transactions on Pattern Analysis and Machine Intelligence: 1-1, which is incorporated herein by reference in its entirety and made a part of the present disclosure.

### 4. Construction of graph convolutional neural network model

This preferred embodiment is based on variants of graph convolutional networks (GCNs): MGC, MGC_F, MGC_S, GINConv, and SageConv. The MGC method differs from GCN in the combination of different graph convolutional layers with distinct propagation rules, demonstrating superior performance compared to other graph neural network methods (see Gligorijević, Vladimir, et al., 2021, "Structure-Based Protein Function Prediction Using Graph Convolutional Networks", Nature Communications 12(1): 3168, which is incorporated herein by reference in its entirety and made a part of the present disclosure).

### 4.1 MGC model

### 4.1.1 Adjacency matrix

The three-dimensional structure of each protein was obtained using prediction software such as the PDB database, AlphaFold2, I-TASSER, RoseTTAFold, Modeller, and Swiss-model. The distances between amino acid residue pairs in the three-dimensional structures were calculated on the basis of the coordinates of the obtained three-dimensional structures of the proteins. In the case that the distance between alpha carbon atoms of two residue pairs is less than 10 angstroms, existence of an edge relationship between the nodes is indicated and recorded as 1, otherwise recorded as 0, with a self-connection recorded as 0, thereby forming the adjacency matrix of the entire protein.

In this embodiment, taking a mutation site as the centre, three amino acids were taken from each side (left and right), resulting in a sequence of 7 consecutive amino acids as the residue sequence. A 7*7 adjacency matrix was then extracted from the adjacency matrix of the entire protein.

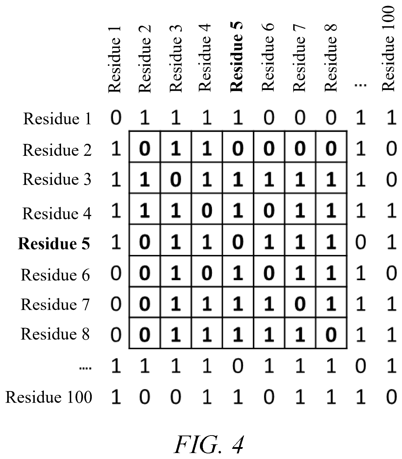

FIG. 4 is a schematic diagram of an adjacency matrix. An adjacency matrix corresponding to a protein with 100 amino acids is shown in the figure. Assuming the mutation site is at residue 5, the boxed area represents the 7*7 adjacency matrix for this residue.

### 4.1.2 Node feature matrix

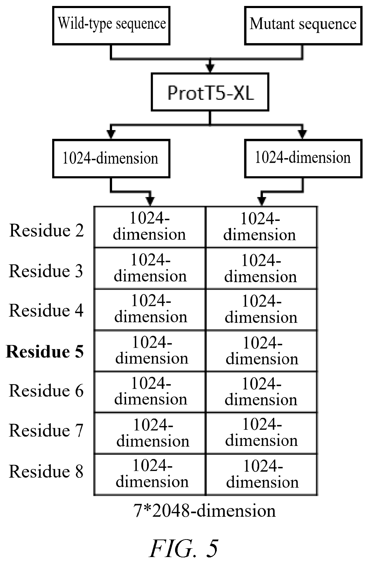

After a piece of protein sequence information was input into the pre-trained model, the model first encoded each amino acid in the protein sequence via an encoder. Each encoded amino acid corresponded to a vector of length 1024; thus, a protein sequence of length N would yield a vector of length N*1024. The model consisted of 24 layers. To acquire the maximum amount of mutation-related information, the last layer was selected as the output. The 1024-dimensional feature vectors corresponding to the mutation sites in the wild-type and mutant proteins were extracted separately, and the feature vectors of the wild-type (i.e., before mutation) and the mutant (i.e., after mutation) were sequentially concatenated into a 2048-dimensional vector. With a sliding window size of 7 and the mutation site as the centre, the three amino acid residues before and after the mutation site were concatenated. Finally, a 7*2048-dimensional feature was obtained as the node feature matrix. The structure is as shown in FIG. 5.

### 4.1.3 Model construction

The matrices generated in Sections 4.1.1 and 4.1.2 were input into the graph convolutional neural network as structural features and sequence features, respectively. Through matrix multiplication and aggregation operations, each layer of the graph network structure updated the node features into 512-dimensional feature vectors. The node features output by the three graph network layers were merged via a residual network structure into a 7*1536-dimensional feature vector (where 1536 = 512*3). Subsequently, an average pooling operation was used to compress the updated feature matrix into a 1*1536-dimensional feature vector, which was used to represent the feature characterization of the sequence of 7 amino acid residues. The specific parameters are as follows: The output dimensions of the three graph convolution layers are 512, 512, and 512, respectively; the number of neurons of the fully connected layer is 256, 2; the optimiser used is Adam; the loss function is binary cross-entropy loss; 'relu' is used as the activation function; 'softmax' is used as the classification function of the output layer; the coefficient of the l2 regularization term is 0.0001, and the dropout rate is 0.5; and the learning rate is 5e-05. It should be understood by those skilled in the art that the average pooling operation here can also be replaced by any pooling operation such as max pooling or K-max pooling.

### 4.2 Construction of MGC_F model

### 4.2.1 First-order adjacency matrix

The adjacency matrix of the entire protein was obtained according to Section 4.1.1. All amino acids spatially within 10 angstroms from the residue at the mutation site were selected with the mutation site residue as the centre, and the adjacency matrix composed of these amino acids was obtained.

### 4.2.2 Node feature matrix

After a piece of protein sequence information was input into the pre-trained model, the model first encoded each amino acid in the protein sequence via an encoder. Each encoded amino acid corresponded to a vector of length 1024; thus, a protein sequence of length N would yield a vector of length N*1024. The model consisted of 24 layers. To acquire the maximum amount of mutation-related information, the last layer was selected as the output in this embodiment. Simultaneously, the 1024-dimensional feature vectors corresponding to the mutation sites in the wild-type and mutant proteins were extracted, and the feature vectors of the wild-type and mutant proteins were sequentially concatenated into a 2048-dimensional vector. Finally, an N_{f}*2048 dimensional feature was obtained as the node feature matrix. N_{f} represents the number of corresponding amino acids obtained from Section 4.2.1, with their order maintained. The number of amino acids obtained may vary among different proteins. Therefore, padding with zeros was performed on the basis of the maximum N_{f}, ensuring that the feature matrices of all proteins had the same size.

### 4.2.3 Model construction

The matrices generated in Sections 4.2.1 and 4.2.2 were input into the graph convolutional neural network as structural features and sequence features, respectively. Through matrix multiplication and aggregation operations, each layer of the graph network structure updated the node features into 512-dimensional feature vectors. The node features output by the three graph network layers were merged via a residual network structure into a N_{f}*1536-dimensional feature vector (where 1536 = 512*3). Subsequently, an average pooling operation was used to compress the updated feature matrix into a 1*1536-dimensional feature vector, which was used to represent the feature characterization of the sequence of N_{f} amino acid residues. The specific parameters are as follows: The output dimensions of the three graph convolution layers are 512, 512, and 512, respectively; the number of neurons of the fully connected layer is 128, 2; the optimiser used is Adam; the loss function is binary cross-entropy loss; 'relu' is used as the activation function; 'softmax' is used as the classification function of the output layer; the coefficient of the l2 regularization term is 0.0005, and the dropout rate is 0.5; and the learning rate is 0.0001. It should be understood by those skilled in the art that the average pooling operation here can also be replaced by any pooling operation such as max pooling or K-max pooling.

### 4.3 Construction of MGC_S model

### 4.3.1 Second-order adjacency matrix

A first-order adjacency matrix was constructed according to the method described in Section 4.2.1. Then, first-order adjacency matrices were recalculated separately for the amino acids in the first-order adjacency matrix. That is, the distances between the amino acid residue pairs were recalculated. In the case that the distance between alpha carbon atoms of two residue pairs is less than 10 angstroms, existence of an edge relationship between the nodes is indicated and recorded as 1, otherwise recorded as 0, with a self-connection recorded as 0. After removing duplicates from all amino acids in the matrices, a new matrix was regenerated, namely the second-order adjacency matrix.

### 4.3.2 Node feature matrix

After a piece of protein sequence information was input into the pre-trained model, the model first encoded each amino acid in the protein sequence via an encoder. Each encoded amino acid corresponded to a vector of length 1024; thus, a protein sequence of length N would yield a vector of length N* 1024. The model consisted of 24 layers. To acquire the maximum amount of mutation-related information, the last layer was selected as the output in this embodiment. Simultaneously, the 1024-dimensional feature vectors corresponding to the mutation site in the wild-type and mutant proteins were extracted, and the feature vectors of the wild-type and mutant proteins were sequentially concatenated into a 2048-dimensional vector. Finally, an Nₛ*2048 dimensional feature was obtained as the node feature matrix. Nₛ represents the number of corresponding amino acids obtained from Section 4.3.1, with their order maintained. The number of amino acids obtained may vary among different proteins. Therefore, zero-padding was performed using the maximum Nₛ as a reference, ensuring that the feature matrices of all proteins had the same size.

### 4.3.3 Model construction

The matrices generated in Sections 4.3.1 and 4.3.2 were input into the graph convolutional neural network as structural features and sequence features, respectively. Through matrix multiplication and aggregation operations, each layer of the graph network structure updated the node features into 512-dimensional feature vectors. The node features output by the three graph network layers were merged via a residual network structure into a Nₛ*1536-dimensional feature vector (where 1536 = 512*3). Subsequently, an average pooling operation was used to compress the updated feature matrix into a 1*1536-dimensional feature vector, which was used to represent the feature characterization of the sequence of Nₛ amino acid residues. This characterization was input into the fully connected layer. The network was composed of a hidden layer with 256 neurons and an output layer with 2 neurons. Finally, the output layer output the probabilities of being predicted as stabilizing mutations and destabilizing mutations. The specific parameters are as follows: The output dimensions of the three graph convolution layers are 512, 512, and 512, respectively; the number of neurons of the fully connected layer is 256, 2; the optimiser used is Adam; the loss function is binary cross-entropy loss; 'relu' is used as the activation function; 'softmax' is used as the classification function of the output layer; the coefficient of the l2 regularization term is 0.0001, and the dropout rate is 0.3; and the learning rate is 5e-05. It should be understood by those skilled in the art that the average pooling operation here can also be replaced by any pooling operation such as max pooling or K-max pooling.

### 4.4 Construction of GINConv model

### 4.4.1 First-order adjacency matrix

Same as the method described in 4.2.1.

### 4.4.2 Node feature matrix

Same as the method described in 4.2.2.

### 4.4.3 Model construction

The matrices generated in Sections 4.4.1 and 4.4.2 were input into the graph convolutional neural network as structural features and sequence features, respectively. Through matrix multiplication and aggregation operations, each layer of the graph network structure updated the node features into 64-dimensional feature vectors. The node features output by the three graph network layers were merged via a residual network structure into a N_{f}*192-dimensional feature vector. Subsequently, an addition pooling operation was applied to compress the updated feature matrix into a 1*192-dimensional feature vector, which was used to represent the feature characterization of the sequence of N_{f} amino acid residues. The specific parameters are as follows: The output dimensions of the three graph convolution layers are 64, 64, and 64, respectively; the number of neurons of the fully connected layer is 64, 64, 2; the aggregator_type of GINConv is 'max'; the initial init_eps value is 0.01; learn_eps is set to False; the optimiser used is Adam; the loss function is binary cross-entropy loss; 'relu' is used as the activation function; 'softmax' is used as the classification function of the output layer; and the learning rate is 0.01.

### 4.5 Construction of SageConv model

### 4.5.1 First-order adjacency matrix

Same as the method described in 4.2.1.

### 4.5.2 Node feature matrix

Same as the method described in 4.2.2.

### 4.5.3 Model construction

The matrices generated in Sections 4.5.1 and 4.5.2 were input into the graph convolutional neural network as structural features and sequence features, respectively. Through matrix multiplication and aggregation operations, each layer of the graph network structure updated the node features into 128-dimensional feature vectors. The node features output by the three graph network layers were merged via a residual network structure into a N_{f}*384-dimensional feature vector. Subsequently, an addition pooling operation was applied to compress the updated feature matrix into a 1*384-dimensional feature vector, which was used to represent the feature characterization of the sequence of N_{f} amino acid residues. The specific parameters are as follows: The output dimensions of the three graph convolution layers are 128, 128, and 128, respectively; the numbers of neurons of each layer in the fully connected layer are 256, 256, 2, respectively; in the SAGEConv model, the aggregator_type is 'gen'; the optimiser used is Adam; the loss function is binary cross-entropy loss; 'relu' is used as the activation function; 'softmax' is used as the classification function of the output layer; and the learning rate is 0.01 (SageConv(128, 256, 'gen', 0.01)).

A summary is provided below.

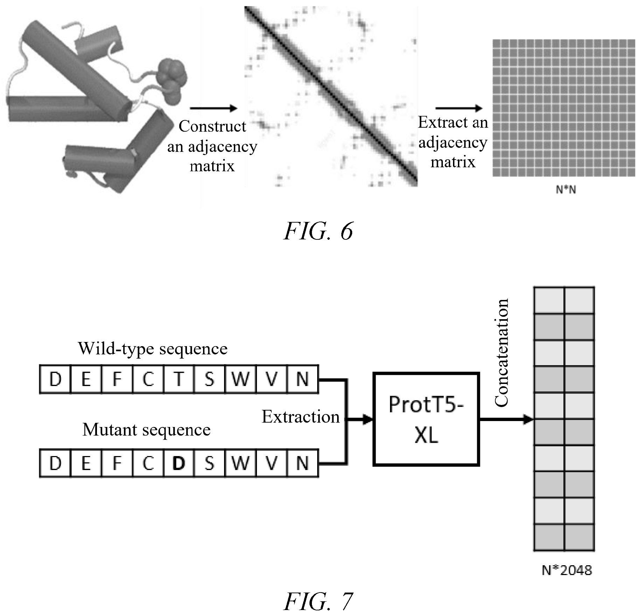

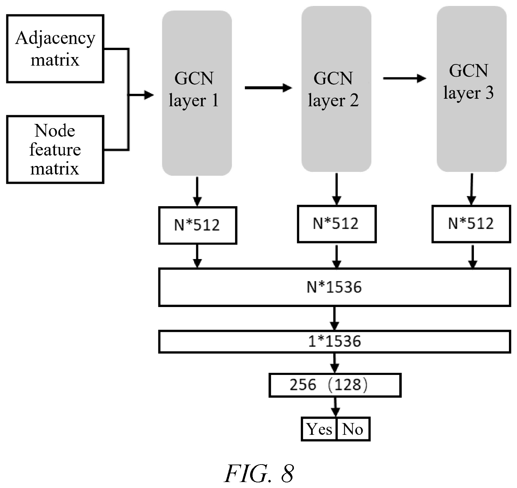

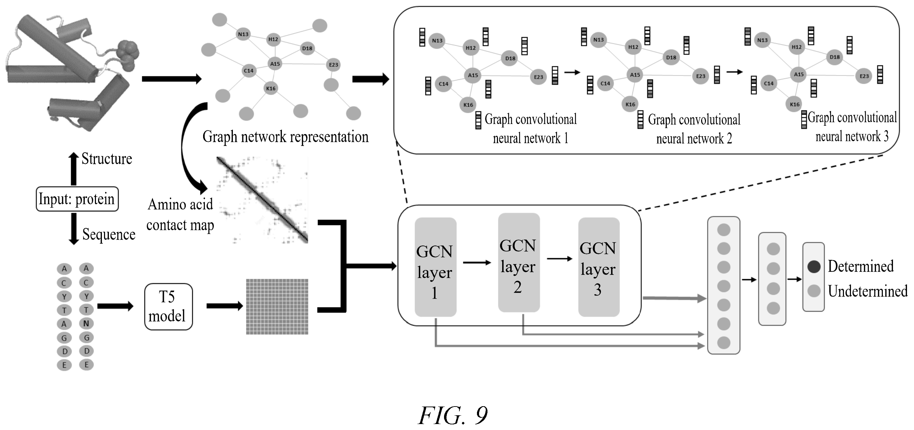

FIG 6 shows a schematic diagram illustrating the structure-based construction of an adjacency matrix. FIG 7 shows a schematic diagram illustrating the sequence-based construction of a node feature matrix. FIG. 8 shows a schematic diagram of the framework of the prediction model. FIG. 9 is a schematic diagram integrating FIGs. 6-8.

On the basis of the above embodiments, it can be seen that in the process of constructing the prediction model, the three-dimensional structural information of the sample protein can be acquired by: acquiring coordinates of a three-dimensional structure of the sample protein; calculating distances between amino acid residue pairs in the three-dimensional structure of the sample protein on the basis of the acquired coordinates of the three-dimensional structure; and when a distance between alpha carbon atoms of two residue pairs is less than 10 angstroms, an edge relationship between the nodes is indicated and recorded as 1, otherwise recorded as 0, with a self-connection recorded as 0, thereby obtaining an adjacency matrix of an entire sample protein. Said obtaining the structural features of the plurality of training samples on the basis of the three-dimensional structural information of the sample protein, may include: taking a mutation site as the centre, extracting an adjacency matrix of a specified size from the adjacency matrix of the entire sample protein, which is used as the structural feature of the sample protein; or recalculating distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between the nodes, recorded as 1, otherwise recorded as 0, with a self-connection recorded as 0, thereby obtaining a second-order adjacency matrix of the protein sample to be predicted. Said training the graph neural network on the basis of the sequence features, the structural features, and the mutation stability labels, so as to obtain the graph neural network-based prediction model, may further include: inputting the sequence features and the structural features into the graph neural network; updating the features through matrix multiplication and aggregation operations using several layers of graph network structures, and then outputting the updated features; merging the features output by the several layers of graph network structures, respectively; compressing the features using a pooling operation; performing stability classification on the features on the basis of a classification function of an output layer; and taking the mutation stability labels as an output target, adjusting network parameters of the graph neural network. The pooling operation described here may be average pooling, max pooling, or K-max pooling.

Correspondingly, in the process of applying the prediction model to protein stability prediction, the three-dimensional structural information of the protein sample to be predicted can be acquired by: acquiring coordinates of a three-dimensional structure of the protein sample to be predicted; calculating distances between amino acid residue pairs in the three-dimensional structure of the protein on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protein sample to be predicted. Said obtaining the structural feature of the protein sample to be predicted on the basis of the three-dimensional structural information of the protein sample to be predicted, includes: taking a mutation site as the centre, extracting an adjacency matrix of a specified size from the adjacency matrix of the entire protein sample to be predicted, which is used as the structural feature of the protein sample to be predicted; or recalculating distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between the nodes, recorded as 1, otherwise recorded as 0, with a self-connection recorded as 0, thereby obtaining a second-order adjacency matrix of the protein sample to be predicted. Said performing prediction using the graph neural network-based prediction model on the basis of the sequence feature and the structural feature, so as to determine the prediction result for stability of the protein sample to be predicted after mutation further includes: inputting the sequence feature and the structural feature into the graph neural network-based prediction model; updating the features through matrix multiplication and aggregation operations using several layers of graph network structures, and then outputting the updated features; merging the features output by the several layers of graph network structures, respectively; compressing the features using an average pooling operation; and performing stability classification on the features on the basis of a classification function of an output layer. Similarly, the pooling operation described here may be average pooling, max pooling, or K-max pooling.

### 5. Performance indicators

In this embodiment, evaluation was performed using the following indicators: area under the curve (AUC), precision, and recall. The specific definitions are as follows:
True Positive (TP)
False Positive (FP)
True Negative (TN)
False Negative (FN)

Among them, TP (True Positive) and TN (True Negative) represent the numbers of correctly classified positive samples and negative samples, respectively, FP (False Positive) and FN (False Negative) represent the numbers of incorrectly classified positive samples and negative samples, respectively, and P and N represent the numbers of positive samples and negative samples, respectively.

AUC is a popular parameter-independent metric used to describe a binary classifier. AUC refers to the area under the receiver operating characteristic curve (ROC), where a larger value indicates better performance of the classifier, with the maximum value being 1.

Precision refers to the proportion of correctly predicted results among all results predicted as positive samples. The calculation formula for precision is as follows: precision = TP/(TP + FP), where TP (True Positive) refers to correctly classified positive samples, and FP (False Positive) refers to incorrectly classified positive samples.

Recall refers to the proportion of the number of correctly predicted positive samples to the total number of positive samples. The calculation formula for recall is as follows: recall = TP/(TP + FN), where FN (False Negative) refers to incorrectly classified negative samples.

### Result evaluation

### 1. Model performance

The model was trained on a self-designed balanced dataset containing 11,834 experimental ΔΔG values of single-point mutations of 328 different proteins from 4 databases. During five-fold cross-validation, the model performance is as shown in Table 1 below.

**Table 1: Performance indicators of the prediction model of the present disclosure on the training set using five-fold cross-validation**

| Method | AUC | Precision | Recall |
|---|---|---|---|
| MGC | 0.87 | 0.80 | 0.79 |
| MGC_F | 0.83 | 0.76 | 0.75 |
| MGC_S | 0.82 | 0.73 | 0.76 |

### 2. Comparison with other methods

To evaluate the differences between the method of the present disclosure and other prediction methods, the model of the present disclosure was compared with other existing state-of-the-art prediction methods on an experimental test set, including: FoldX (see Guerois, Raphael, Jens Erik Nielsen and Luis Serrano, 2002, "Predicting Changes in the Stability of Proteins and Protein Complexes: A Study of More than 1000 Mutations", Journal of Molecular Biology 320(2): 369-87, which is incorporated herein by reference in its entirety and made a part of the present disclosure), Rosetta (Leman, Julia Koehler et al., 2020, "Macromolecular Modeling and Design in Rosetta: Recent Methods and Frameworks", Nature Methods 17(7): 665-80, which is incorporated herein by reference in its entirety and made a part of the present disclosure), Thermnet (Ofoegbu, Tochukwu C. et al., 2019, "PhyreRisk: A Dynamic Web Application to Bridge Genomics, Proteomics and 3D Structural Data to Guide Interpretation of Human Genetic Variants", Journal of Molecular Biology 431(13): 2460-66, which is incorporated herein by reference in its entirety and made a part of the present disclosure), PremPS (Chen, Yuting et al., 2020, "PremPS: Predicting the Impact of Missense Mutations on Protein Stability", PLoS computational biology 16(12): e1008543, which is incorporated herein by reference in its entirety and made a part of the present disclosure), I-Mutant (Capriotti, E., P. Fariselli and R. Casadio, 2005, "I-Mutant2.0: Predicting Stability Changes upon Mutation from the Protein Sequence or Structure", Nucleic Acids Research 33(Web Server): W306-10, which is incorporated herein by reference in its entirety and made a part of the present disclosure), and DDGun (Montanucci, Ludovica et al., 2022, "DDGun: An Untrained Predictor of Protein Stability Changes upon Amino Acid Variants", Nucleic Acids Research 50(W1): W222-27, which is incorporated herein by reference in its entirety and made a part of the present disclosure). None of the samples in this experimental test set appeared in the training sets of the method of the present disclosure and the above prediction methods. The results are as shown in Table 2 below.

**Table 2: Performance comparison between the model of the present disclosure and other methods on the experimental test set Tm141**

| Method | AUC | Precision | Recall |
|---|---|---|---|
| MGC | 0.88 | 0.76 | 0.67 |
| MGC_F | 0.89 | 0.73 | 0.67 |
| MGC_S | 0.87 | 0.72 | 0.66 |
| GINConv | 0.86 | 0.71 | 0.62 |
| SageConv | 0.86 | 0.59 | 0.54 |
| PremPS | 0.88 | 0.58 | 0.29 |
| DDGun | 0.77 | 0.34 | 0.50 |
| I-Mutant | 0.86 | 0.60 | 0.50 |
| FoldX | 0.81 | 0.52 | 0.71 |
| Rosetta | 0.80 | 0.50 | 0.50 |
| Thermnet | 0.81 | 0.33 | 0.79 |

As can be seen from the table above, the graph-based method has a significant advantage in precision compared with previous methods, while maintaining a high recall.

**Table 3: Performance comparison between the model of the present disclosure and other methods on the experimental test set S669**

| Method | AUC | Precision | Recall |
|---|---|---|---|
| MGC | 0.78 | 0.57 | 0.48 |
| MGC_F | 0.78 | 0.55 | 0.45 |
| GINConv | 0.80 | 0.57 | 0.58 |
| SageConv | 0.79 | 0.56 | 0.51 |
| MGC_S | 0.75 | 0.60 | 0.39 |
| PremPS | 0.64 | 0.43 | 0.23 |
| DDGun | 0.71 | 0.43 | 0.42 |
| I-Mutant | 0.66 | 0.48 | 0.21 |
| FoldX | 0.62 | 0.34 | 0.41 |
| Rosetta | 0.64 | 0.46 | 0.25 |
| Thermnet | 0.68 | 0.36 | 0.51 |

The results show that the performance of the method of the present disclosure on the test set is outstanding as compared with other methods.

### 3. Experimental validation of prediction model

To verify the practical application of the model of the present disclosure in experiments, additional validation was performed by predicting existing experimental results in the literature. The results are as shown in Table 4 below:

**Table 4: Test results of the method of the present disclosure in practical applications**

| Protein (Uniprot) | Mutation site | Prediction result | Experimental results | Reference (PMID) |
|---|---|---|---|---|
| P03050 | Q39L | Increased stability | Increased stability | 10048325 |
| P13123 | W24A | Decreased stability | Decreased stability | 15654747 |

### Prediction process

Here, two scenarios were taken as examples to describe the process of using the prediction model to perform prediction of protein stability.

One scenario was that the input protein sequence information included a single piece of protein sequence and mutation site information. First, on the basis of the input single piece of protein sequence and mutation site information, protein mutation site preprocessing was performed. Specifically, the input protein sequence was recorded as an original sequence, and a sequence after mutation was obtained on the basis of the mutation site information. Then, amino acid embedding layer extraction was performed, which was the sequence information extraction described above. That is, the original sequence was input into a natural language processing T5 pre-trained model to extract embedding features; the protein sequence after mutation was input into a natural language processing T5 pre-trained model to extract embedding features; amino acid embedding features were extracted on the basis of the mutation site, and the features before and after mutation were concatenated as an input for the subsequent model. Next, a graph network was constructed, which required a protein embedding layer feature network and a protein residue interaction feature network. The above feature networks were applied to an MGC prediction model to predict the change in protein thermal stability before and after amino acid mutation. Finally, the prediction results were output and displayed. For the scenario exemplified here, the output prediction results for single-point mutations could be output.

Another scenario was that the input protein sequence information only included a single protein sequence. First, on the basis of the input single protein sequence, protein mutation site preprocessing was performed. Specifically, the input protein sequence was recorded as an original sequence, and the amino acid at each site in the protein sequence was sequentially mutated to each of the other 19 types of amino acids, with each resulting sequence recorded as a sequence after mutation. Then, amino acid embedding layer extraction was performed, which was the sequence information extraction described above. That is, the original sequence was input into a natural language processing T5 pre-trained model to extract embedding features; the protein sequence after mutation was input into a natural language processing T5 pre-trained model to extract embedding features; amino acid embedding features were extracted on the basis of the mutation site, and the features before and after mutation were concatenated as an input for the subsequent model. Next, a graph network was constructed, which required a protein embedding layer feature network and a protein residue interaction feature network. The above feature networks were applied to an MGC prediction model to predict the change in protein thermal stability before and after amino acid mutation. Finally, the prediction results were output and displayed. For the scenario exemplified here, the final prediction results could be output as a table of all stabilizing mutation probabilities and/or a ranking of all stabilizing mutation possibilities.

### Conclusion

More stable protein mutants are essential for the academic and industrial sectors to explore additional applications of these proteins. The present disclosure proposes a new method for identifying the impact of amino acid mutations on protein stability. The method of the present disclosure is based on an advanced natural language processing model combined with a graph neural network, can be applied to various biological fields, and exhibits superior performance to earlier methods. This method has been successfully applied to predict stability changes caused by mutations in multiple experiments, demonstrating the applicability of the method of the present disclosure in protein engineering.

Discussion on the advantages of the method of the present disclosure over the prior art is provided below.

As can be seen from the above description:
1. The method of the present disclosure is based on the latest existing natural language processing model ProtT5-XL combined with structure-based features, and has been successfully applied to protein stability prediction, achieving results superior to those of the prior art, which have been verified by experiments.
2. The method of the present disclosure is based on a graph neural network, which significantly enhances the prediction performance of the model.
3. The training set for the method of the present disclosure is collected from a variety of the latest databases, with a sample size nearly twice that of previous ones.
4. After inversion, not only the training dataset used in the method of the present disclosure is expanded, but also the data of stabilizing mutations and destabilizing mutations are balanced, ensuring the predictive robustness of the model.

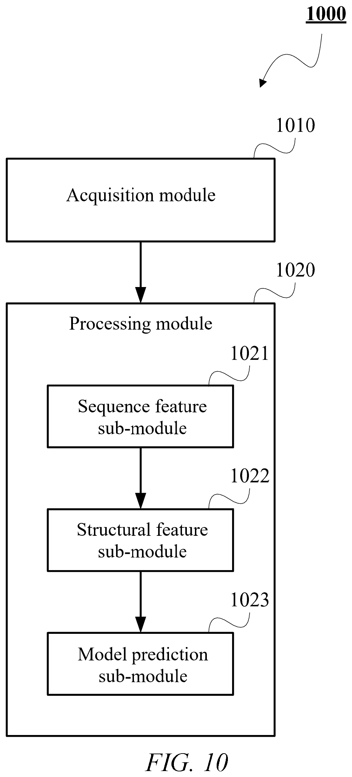

It should be understood by those skilled in the art that on the basis of the prediction method for stability of the protein after mutation of the present disclosure, a system for predicting stability of a protein after mutation can be developed. FIG. 10 shows a schematic block diagram of a system for predicting stability of a protein after mutation according to the present disclosure. Specifically, a system 1000 for predicting stability of a protein after mutation includes an acquisition module 1010 and a processing module 1020.

The acquisition module 1010 is configured to acquire sequence information and three-dimensional structural information of a protein sample to be predicted.

The processing module 1020 is configured to obtain a prediction result for stability of the protein sample to be predicted after mutation by inputting the sequence information and the three-dimensional structural information of the protein sample to be predicted. As shown in FIG. 10, the processing module 1020 may further include: a sequence feature sub-module 1021, configured to perform feature extraction using a pre-trained model on the basis of sequence information of the protein sample to be predicted, so as to obtain a sequence feature of the protein sample to be predicted; a structural feature sub-module 1022, configured to obtain a structural feature of the protein sample to be predicted on the basis of three-dimensional structural information of the protein sample to be predicted; and a model prediction sub-module 1023, configured to perform prediction using a graph neural network-based prediction model on the basis of the sequence feature and the structural feature, so as to determine a prediction result for stability of the protein sample to be predicted after mutation.

It should be understood by those skilled in the art that the system for predicting stability of the protein after mutation can be implemented by a computer. For example, the following operations can be implemented by executing a computer program: acquiring sequence information and three-dimensional structural information of a protein sample to be predicted; and obtaining a prediction result for stability of the protein sample to be predicted after mutation by inputting the sequence information and the three-dimensional structural information of the protein sample to be predicted, where the operation further includes the following sub-operations: performing feature extraction using a pre-trained model on the basis of the sequence information of the protein sample to be predicted, so as to obtain a sequence feature of the protein sample to be predicted; obtaining a structural feature of the protein sample to be predicted on the basis of the three-dimensional structural information of the protein sample to be predicted; and performing prediction using a graph neural network-based prediction model on the basis of the sequence feature and the structural feature, so as to determine a prediction result for stability of the protein sample to be predicted after mutation.

That is, although in the graph neural network-based system for predicting stability of the protein according to the present disclosure, operational functions corresponding to the prediction method are described as modules or sub-modules, it should be understood by those skilled in the art that such modules or sub-modules may be circuit components or other physical assemblies, or they may not be composed of circuit components or other physical assemblies, but rather functional modules constructed through computer programs.

In addition, it should be recognised by those skilled in the art that the method of the present disclosure can be implemented as a computer program. As described above in conjunction with the accompanying drawings, the method of the above embodiment is executed by one or more programs, and instructions in the programs cause a computer or processor to execute the algorithm described in conjunction with the accompanying drawings. These programs can be stored using various types of non-transitory computer-readable media and provided to a computer or processor. Non-transitory computer-readable media include various types of tangible storage media. Examples of non-transitory computer-readable media include magnetic recording media (such as floppy disks, magnetic tapes, and hard disk drives), magneto-optical recording media (such as magneto-optical disks), CD-ROMs (Compact Disc Read-Only Memories), CD-R, CD-R/W, and semiconductor memories (such as ROMs (Read-Only Memories), PROMs (Programmable ROMs), EPROMs (Erasable Programmable ROMs), flash ROMs, and RAMs (Random Access Memory)). Further, these programs can be provided to a computer using various types of transitory computer-readable media. Examples of transitory computer-readable media include electrical signals, optical signals, and electromagnetic waves. Transitory computer-readable media can be configured to provide programs to a computer via wired communication paths such as electric wires and optical fibers, or wireless communication paths.

For example, according to an embodiment of the present disclosure, a non-transitory computer-readable storage medium may be provided for storing a computer program, where the computer program includes instructions which, when executed by a processor of an electronic device, cause the electronic device to perform the above-described method for predicting stability of the protein after mutation.

In addition, according to the present disclosure, a computer system may also be provided. The computer system includes: a processor; a memory; and a computer program. The computer program is stored in the memory and configured to be executed by the processor. The computer program includes instructions for performing the above-described method for predicting stability of the protein after mutation.

In another aspect, according to an embodiment of the present disclosure, a non-transitory computer-readable storage medium may be provided for storing a computer program, where the computer program includes instructions which, when executed by a processor of an electronic device, cause the electronic device to perform the above-described construction method for the model for predicting stability of the protein after mutation.

In addition, according to the present disclosure, a computer system may also be provided. The computer system includes: a processor; a memory; and a computer program. The computer program is stored in the memory and configured to be executed by the processor. The computer program includes instructions for performing the above-described construction method for the model for predicting stability of the protein after mutation.

The implementations of the present disclosure are not limited to those described in the above embodiments. Without departing from the spirit and scope of the present disclosure, those of ordinary skill in the art can make various changes and improvements to the present disclosure in form and detail, and all such changes and improvements are deemed to fall within the scope of protection of the present disclosure.

## Claims

1. A method for predicting stability of a protein after mutation, **characterized in that** the method comprises:
performing feature extraction using a pre-trained model on the basis of sequence information of a protein sample to be predicted, so as to obtain a sequence feature of the protein sample to be predicted;
obtaining a structural feature of the protein sample to be predicted on the basis of three-dimensional structural information of the protein sample to be predicted; and
performing prediction using a graph neural network-based prediction model on the basis of the sequence feature and the structural feature, so as to determine a prediction result for stability of the protein sample to be predicted after mutation.

2. The method according to claim 1, **characterized in that** the sequence information of the protein sample to be predicted comprises amino acid sequence information of the protein sample to be predicted before mutation and after mutation.

3. The method according to claim 1, **characterized in that** the sequence information of the protein sample to be predicted comprises amino acid sequence information of the protein sample to be predicted before mutation and specified mutation information.

4. The method according to claim 3, **characterized in that** the method further comprises: obtaining amino acid sequence information of the protein sample to be predicted after mutation on the basis of the amino acid sequence information of the protein sample to be predicted before mutation and the specified mutation information.

5. The method according to claim 3, **characterized in that** the specified mutation information comprises a specified mutation site and/or a specified amino acid type after mutation.

6. The method according to claim 2 or 4, **characterized in that** said performing feature extraction using the pre-trained model on the basis of the sequence information of the protein sample to be predicted, so as to obtain the sequence feature of the protein sample to be predicted, further comprises:
performing feature extraction separately on the amino acid sequence information of the protein sample to be predicted before mutation and after mutation using the pre-trained model, so as to obtain sequence feature information before mutation and sequence feature information after mutation; and
concatenating the sequence feature information before mutation and the sequence feature information after mutation, so as to obtain the sequence feature of the protein sample to be predicted.

7. The method according to claim 1, **characterized in that** the pre-trained model is implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

8. The method according to claim 1, **characterized in that** the three-dimensional structural information of the protein sample to be predicted comprises three-dimensional structural information of the protein sample to be predicted acquired through at least one of the following databases or prediction software: PDB database, AlphaFold2, I-TASSER, RoseTTAFold, Modeller, and Swiss-model.

9. The method according to claim 1, **characterized in that** the three-dimensional structural information comprises an interaction network, a secondary structure, an amino acid residue distance, or a physical environment; preferably, the three-dimensional structural information comprises an interaction network.

10. The method according to claim 9, **characterized in that** the three-dimensional structural information of the protein sample to be predicted is acquired by:
acquiring coordinates of a three-dimensional structure of the protein sample to be predicted;
calculating distances between amino acid residue pairs in the three-dimensional structure of the protein on the basis of the acquired coordinates of the three-dimensional structure; and
when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protein sample to be predicted.

11. The method according to claim 10, **characterized in that** said obtaining the structural feature of the protein sample to be predicted on the basis of the three-dimensional structural information of the protein sample to be predicted, comprises:
taking a mutation site as the centre, extracting an adjacency matrix of a specified size from the adjacency matrix of the entire protein sample to be predicted, which is used as the structural feature of the protein sample to be predicted; or
recalculating the distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between nodes, recorded as 1, otherwise recorded as 0, with a self-connection recorded as 0, thereby obtaining a second-order adjacency matrix of the protein sample to be predicted.

12. The method according to claim 1, **characterized in that** said performing prediction using the graph neural network-based prediction model on the basis of the sequence feature and the structural feature, so as to determine the prediction result for stability of the protein sample to be predicted after mutation, further comprises:
inputting the sequence feature and the structural feature into the graph neural network-based prediction model;
updating the features through matrix multiplication and aggregation operations using several layers of graph network structures, and then outputting the updated features;
merging the features output by the several layers of graph network structures, respectively;
compressing the features using a pooling operation; and
performing stability classification on the features on the basis of a classification function of an output layer.

13. The method according to claim 1, **characterized in that** the graph neural network-based prediction model is constructed through the steps of:
acquiring a training set, wherein the training set comprises information of a plurality of training samples, and the information of each of the training samples comprises three-dimensional structural information of a sample protein, amino acid sequence information of the sample protein before mutation and after mutation, and a mutation stability label;
performing feature extraction using a pre-trained model on the basis of the amino acid sequence information of the sample protein before mutation and after mutation, so as to obtain sequence features of the plurality of training samples;
obtaining structural features of the plurality of training samples on the basis of the three-dimensional structural information of the sample protein; and
training a graph neural network on the basis of the sequence features, the structural features, and the mutation stability labels, so as to obtain the graph neural network-based prediction model.

14. The method according to claim 13, **characterized in that** the graph neural network-based prediction model is a classifier.

15. The method according to claim 13, **characterized in that** the graph neural network is a graph convolutional network.

16. The method according to claim 15, **characterized in that** the graph neural network-based prediction model comprises one of the following models: MGC model, MGC_F model, MGC_S model, GINConv model, and SageConv model.

17. The method according to claim 13, **characterized in that** the step of acquiring the training set comprises:
acquiring experimental data from a public database as a training set; and
doubling the size of the training set by performing inversion processing on the experimental data from the public database.

18. A construction method for a prediction model for stability of a protein after mutation, **characterized in that** the method comprises:
acquiring a training set, wherein the training set comprises information of a plurality of training samples, and the information of each of the training samples comprises three-dimensional structural information of a sample protein, amino acid sequence information of the sample protein before mutation and after mutation, and a mutation stability label;
performing feature extraction using a pre-trained model on the basis of the amino acid sequence information of the sample protein before mutation and after mutation, so as to obtain sequence features of the plurality of training samples;
obtaining structural features of the plurality of training samples on the basis of the three-dimensional structural information of the sample protein; and
training a graph neural network on the basis of the sequence features, the structural features, and the mutation stability labels, so as to obtain a graph neural network-based prediction model.

19. The method according to claim 18, **characterized in that** said performing feature extraction using the pre-trained model on the basis of the amino acid sequence information of the sample protein before mutation and after mutation, so as to obtain the sequence features of the plurality of training samples, comprises:
performing feature extraction separately on the amino acid sequence information of the sample protein before mutation and after mutation using the pre-trained model, so as to obtain sequence feature information before mutation and sequence feature information after mutation; and
concatenating the sequence feature information before mutation and the sequence feature information after mutation, so as to obtain the sequence features of the plurality of training samples.

20. The method according to claim 18, **characterized in that** the pre-trained model is implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

21. The method according to claim 18, **characterized in that** the three-dimensional structural information of the sample protein comprises three-dimensional structural information of the sample protein acquired through at least one of the following databases or prediction software: PDB database, AlphaFold2, I-TASSER, RoseTTAFold, Modeller, and Swiss-model.

22. The method according to claim 18, **characterized in that** the three-dimensional structural information comprises an interaction network, a secondary structure, an amino acid residue distance, or a physical environment; preferably, the three-dimensional structural information comprises an interaction network.

23. The method according to claim 22, **characterized in that** the three-dimensional structural information of the sample protein is acquired by:
acquiring coordinates of a three-dimensional structure of the sample protein;
calculating distances between amino acid residue pairs in the three-dimensional structure of the sample protein on the basis of the acquired coordinates of the three-dimensional structure; and
when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire sample protein.

24. The method according to claim 23, **characterized in that** said obtaining the structural features of the plurality of training samples on the basis of the three-dimensional structural information of the sample protein, comprises:
taking a mutation site as the centre, extracting an adjacency matrix of a specified size from the adjacency matrix of the entire sample protein, which is used as the structural feature of the sample protein; or
recalculating the distances between amino acid residue pairs on the basis of amino acids in the adjacency matrix of the entire protein sample to be predicted, and when the distance between alpha carbon atoms of two residue pairs is less than 10 angstroms, indicating existence of an edge relationship between nodes, recorded as 1, otherwise recorded as 0, with a self-connection recorded as 0, thereby obtaining a second-order adjacency matrix of the protein sample to be predicted.

25. The method according to claim 18, **characterized in that** said training the graph neural network on the basis of the sequence features, the structural features, and the mutation stability labels, so as to obtain the graph neural network-based prediction model, comprises:
inputting the sequence features and the structural features into the graph neural network;
updating the features through matrix multiplication and aggregation operations using several layers of graph network structures, and then outputting the updated features;
merging the features output by the several layers of graph network structures, respectively;
compressing the features using a pooling operation;
performing stability classification on the features on the basis of a classification function of an output layer; and
taking the mutation stability labels as an output target, adjusting network parameters of the graph neural network.

26. The method according to claim 18, **characterized in that** the graph neural network-based prediction model is a classifier.

27. The method according to claim 18, **characterized in that** the graph neural network is a graph convolutional network.

28. The method according to claim 27, **characterized in that** the graph neural network-based prediction model comprises one of the following models: MGC model, MGC_F model, MGC_S model, GINConv model, and SageConv model.

29. The method according to claim 18, **characterized in that** the step of acquiring the training set comprises:
acquiring experimental data from a public database as a training set; and
doubling the size of the training set by performing inversion processing on the experimental data from the public database.

30. A system for predicting stability of a protein after mutation, **characterized in that** the system comprises:
an acquisition module, configured to acquire sequence information and three-dimensional structural information of a protein sample to be predicted; and
a processing module, configured to obtain a prediction result for stability of the protein sample to be predicted after mutation by inputting the sequence information and the three-dimensional structural information of the protein sample to be predicted, wherein the processing module further comprises the following sub-modules:
a sequence feature sub-module, configured to perform feature extraction using a pre-trained model on the basis of the sequence information of the protein sample to be predicted, so as to obtain a sequence feature of the protein sample to be predicted;
a structural feature sub-module, configured to obtain a structural feature of the protein sample to be predicted on the basis of the three-dimensional structural information of the protein sample to be predicted; and
a model prediction sub-module, configured to perform prediction using a graph neural network-based prediction model on the basis of the sequence feature and the structural feature, so as to determine a prediction result for stability of the protein sample to be predicted after mutation.

31. A computer-implemented system for predicting stability of a protein after mutation, **characterized in that** the computer-implemented system implements the following operations by executing a computer program:
acquiring sequence information and three-dimensional structural information of a protein sample to be predicted; and
obtaining a prediction result for stability of the protein sample to be predicted after mutation by inputting the sequence information and the three-dimensional structural information of the protein sample to be predicted, wherein the operation further comprises the following sub-operations:
performing feature extraction using a pre-trained model on the basis of the sequence information of the protein sample to be predicted, so as to obtain a sequence feature of the protein sample to be predicted;
obtaining a structural feature of the protein sample to be predicted on the basis of the three-dimensional structural information of the protein sample to be predicted; and
performing prediction using a graph neural network-based prediction model on the basis of the sequence feature and the structural feature, so as to determine a prediction result for stability of the protein sample to be predicted after mutation.

32. A non-transitory computer-readable storage medium for storing a computer program, **characterized in that** the computer program comprises instructions which, when executed by a processor of an electronic device, cause the electronic device to perform the method for predicting stability of the protein after mutation according to any one of claims 1-17, or the construction method for the prediction model for stability of the protein after mutation according to any one of claims 18-29.

33. A computer system, **characterized in that** the method comprises:
a processor;
a memory; and
a computer program, wherein the computer program is stored in the memory and configured to be executed by the processor, and the computer program comprises instructions for performing the method for predicting stability of the protein after mutation according to any one of claims 1-17, or the construction method for the prediction model for stability of the protein after mutation according to any one of claims 18-29.
